# EUROPEAN PATENT APPLICATION

(11) **EP 1 338 895 A1**
(43) Date of publication of application: **27.08.2003**
(21) Application number: 02251186.9
(22) Date of filing: 21.02.2002
(51) Int. Cl.: G01N 33/543, G01N 33/68

(54) **High density allergen microarray**

(71) Applicant: Co-Wealth Medical Science & Biotechnology, Inc., Taipei (TW)
(72) Inventor: Duen, Lee, Taipei 106 (TW); Chuan, Chin, Pinchen City, Taoyuan Country (TW)
(74) Representative: Smaggasgale, Gillian Helen

(57) **Abstract**

The present invention discloses an allergen detection chip, which comprises a solid substrate, and at least on e allergen fastened to said solid substrate, wherein said solid substrate comprises (i) a substrate layer; (ii) an adhesive intermediate layer located on said substrate layer; and (iii) a surface layer located on said adhesive intermediate layer. The present invention also discloses a method for preparing the abovementioned allergen detection chip, a kit containing said allergen detection chip and its application in detecting various allergens IgE and IgG antibody in a sample.

## Description

### Background of the Invention

### Field of the Invention

The present invention relates to an allergen detection chip, its preparation method, a kit containing said allergen detection chip and its application in detecting various allergens IgE and/or IgG antibody in a sample.

### Description of the Related Art

An allergic reaction is a clinical symptom, e.g. eruption, rubefaction, itchiness, nausea, vomiting, inappetence, sniveling, rhinitis, asthma, dyspnea, fever, arthralgia, shock, etc., which is caused by a foreign material, generally known as an allergen, that has entered a human body through the skin, the respiratory tract or the alimentary canal, and stimulated the immune mechanism of the human body. In 1964, Gell and Coombs ("Clinical Configurations of Immunology", Blackwell, Oxford, 1963), based on the speed of an allergic reaction and the mechanism of the reaction, classified the allergic reactions into the following four types:
(1) First type: immediate, IgE-mediated hypersensitive;
(2) Second type: antibody dependent cytotoxicity hypersensitive;
(3) Third type: immuno-complex-mediated hypersensitive; and
(4) Fourth type: delayed T cell-mediated hypersensitive.

An ordinary allergic reaction belongs to the first type or the third type. However, there are occasions where one symptom is caused by a combination of several allergic reactions. Common allergies, e.g. pollinosis, rhinallergosis, measles, asthma, etc., belong to the first type; and some chronic allergic reactions, e.g. Arthus reaction and lupus erythematosus, belong to the third type.

Except the first type of allergic reactions can be treated by a reduction on the allergic reaction, at present, the other types of the allergic reactions can only be reduced of the clinical symptoms by the use of medicines. Therefore, identifying the allergens that trigger a patient's allergic reaction and avoiding the contact of the allergens by the patient are the most effective ways of improving over an anaphylaxis.

Generally speaking, current detection on the allergen mainly comprises a dermal sensitivity test and a serum IgE antibody test. The former test comprises injecting an allergen at a low concentration and a low dosage to the subcutaneous tissue for observing the allergic reaction thereof. Even though the dermal sensitivity test has a high accuracy and a low cost, it requires many subcutaneous injections which might cause a person under test feeling uncomfortable and fear. Furthermore, there exists the possibility of generating a false positive reaction and causing an error judgement. Moreover, since the allergen is directly injected, there exists the danger of causing an allergic shock. A serum IgE antibody test uses an enzyme-linked immunization sorption analysis (ELISA), and detects the IgE antibody of various allergens in the serum and their concentrations. Although, this method can detect many allergens in one test, it requires a large quantity of specimen and test material, thereby causing a high cost and unsuitable for popular use.

The technique of a biochip combines techniques in various fields which include molecular biology, biochemistry, material chemistry, microelectronics, surface chemistry, photoelectric science, and biological information science, etc. The technique of a biochip enables the continuation and miniaturization of the detection and analysis of a sample, and tests the nucleic acid, proteins, cells and other biological components rapidly, accurately and in large quantities. The current biochips are based on gene chips, which include mRNA chips, cDNA chips, microPCR chips, etc. However, no protein chip is available for a direct application on the clinical test and diagnosis, particularly a chip for testing the allergens which cause allergic reactions.

Therefore, the field of medical test technique is in an urgent need of developing a method for testing the allergens rapidly, accurately, in great quantities, versatilely, at a low specimen use, with a high sensitivity, and at a low cost.

### Summary of the Invention

Accordingly, the present invention combines a protein chip with an antigen-antibody specificity, and prepares an allergen test chip which not only can be equal to an enzyme-linked immunization sorption analysis (ELISA) and able to perform a large amount of screening in a short period of time, but also can greatly reduce the amount of specimen needed while achieving the abovementioned requirements.

A first configuration of the present invention provides an allergen test chip, which comprises a solid substrate, and at least one allergen fastened to said solid substrate, wherein said solid substrate comprises (i) a substrate layer; (ii) an adhesive intermediate layer located on said substrate layer; and (iii) a surface layer located on said adhesive intermediate layer.

In another embodiment, the solid substrate per se can provide the supporting function, while combining with the allergen. Therefore, the present invention also provides an allergen test chip, which comprises (i) a solid substrate; and (ii) at least one allergen fastened to said solid substrate.

A second configuration of the present invention provides a method for preparing an allergen test chip, which comprises (a) coating an adhesive intermediate layer on a substrate layer; (b) coating a surface layer on said adhesive intermediate layer; and (c) fastening at least one allergen to said surface layer.

In another embodiment, the solid substrate per se can also provide a supporting function, while combining with the allergen. Therefore, the present invention also provides a method for preparing an allergen test chip, which comprises fastening at least one allergen to a solid substrate.

A third configuration of the present invention provides a kit for testing an allergen, which comprises (i) anyone of the abovementioned allergen test chip; (ii) an isolation solution for isolating a portion of said test chip not in combination with the allergen; (iii) a secondary antibody which can specifically combine with an anti-allergic antibody; and (iv) signal generation means for generating a signal by operatively in combination with said secondary antibody.

A fourth configuration of the present invention provides a method for testing an allergen, which comprises (a) providing anyone of the abovementioned allergen test chip; (b) using an isolation solution to isolate a portion of said test chip not in combination with said allergen; (c) contacting the serum sample of a patient with said test chip; (d) using a washing solution to perform a washing step; (e) providing a signal generation means capable of generating a signal by operatively combining with a secondary antibody, wherein said secondary antibody can specifically combine with an anti-allergic antibody; and (f) measuring the signal generated by said signal generation means.

In order to further elaborate the objectives, characteristics and merits of the present invention, preferred embodiments, together with figures, are described in detail in the following context.

### Brief Description of the Drawings

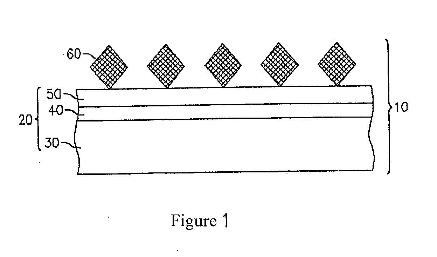

Fig. 1 is a schematic diagram of an allergen test chip according to the present invention.

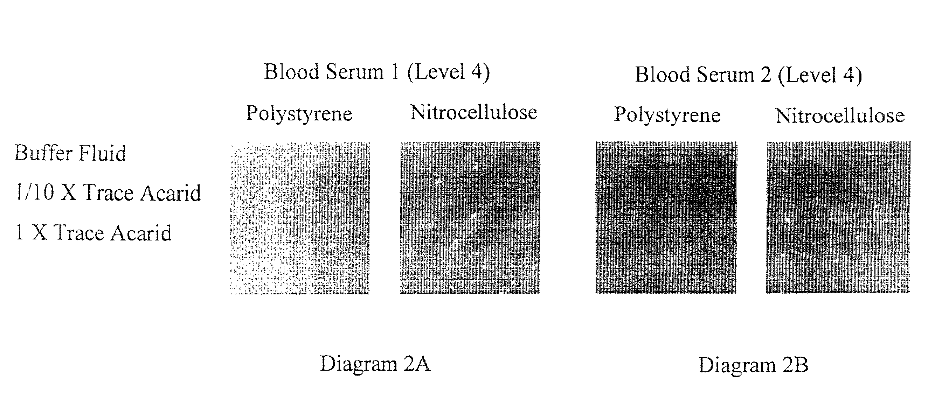

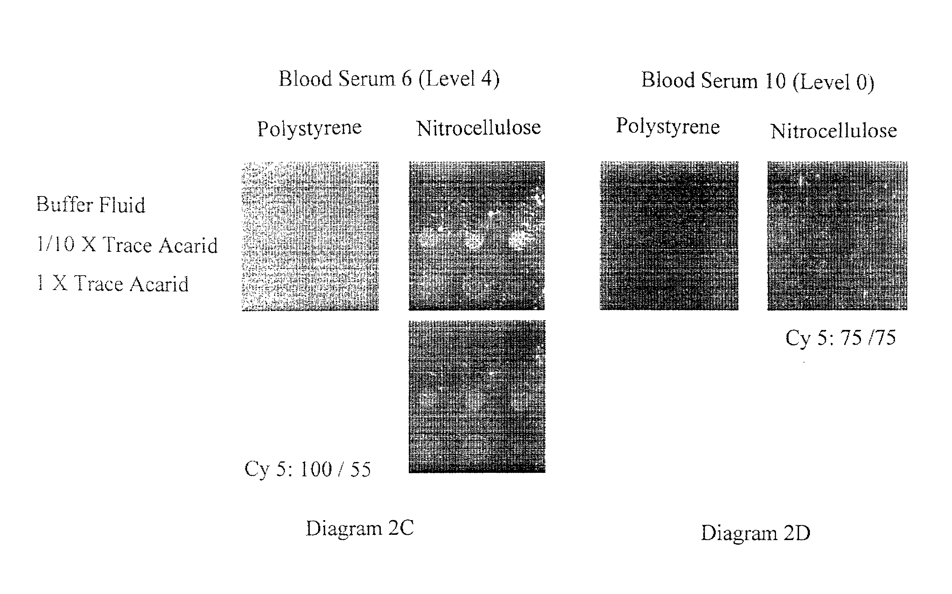

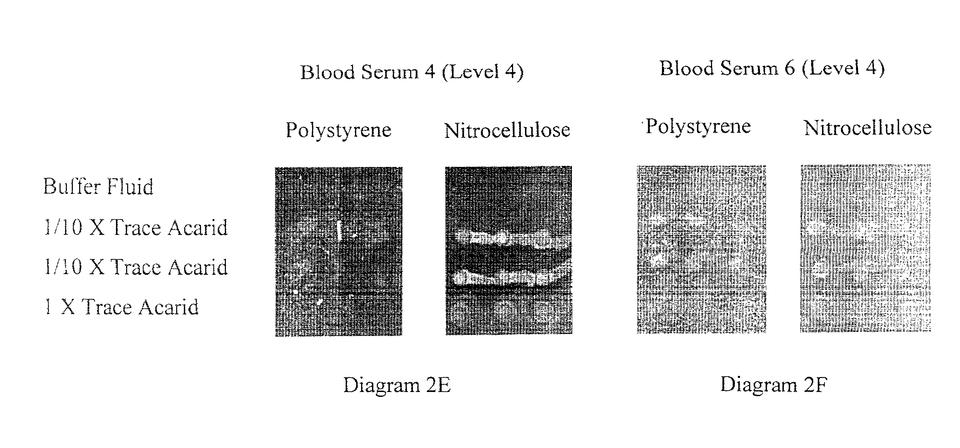

Figs. 2(A)-(J) are the results measured by an allergen chip of dust mite; Fig. 2A: serum 1 (fourth grade); polystyrene; nitrocellulose; buffer solution; 1/10X dust mite; 1X dust mite; Fig. 2B: serum 2 (fourth grade); polystyrene; nitrocellulose; Fig. 2C: serum 6 (fourth grade); polystyrene; nitrocellulose; buffer solution; 1/10X dust mite; 1X dust mite; Fig. 2D: serum 10 (0th grade); polystyrene; nitrocellulose; Fig. 2E: serum 4 (fourth grade); polystyrene; nitrocellulose; buffer solution; 1/10X dust mite; 1/10X dust mite; 1X dust mite; Fig. 2F: serum 6 (fourth grade); polystyrene; nitrocellulose; Fig. 2G: serum 5 (third grade); polystyrene; nitrocellulose; buffer solution; 1/10X dust mite; 1/10X dust mite; 1X dust mite; Fig. 2H: serum 7 (third grade); polystyrene; nitrocellulose; Fig. 21: serum 10 (0th grade); polystyrene; nitrocellulose; buffer solution; 1/10X dust mite; 1/10X dust mite; 1X dust mite; Fig. 2J: PBS; polystyrene; nitrocellulose.

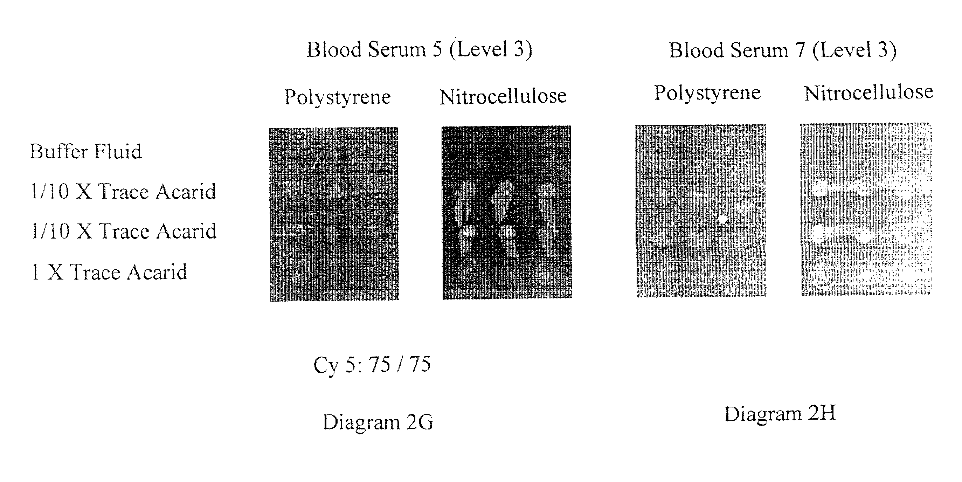

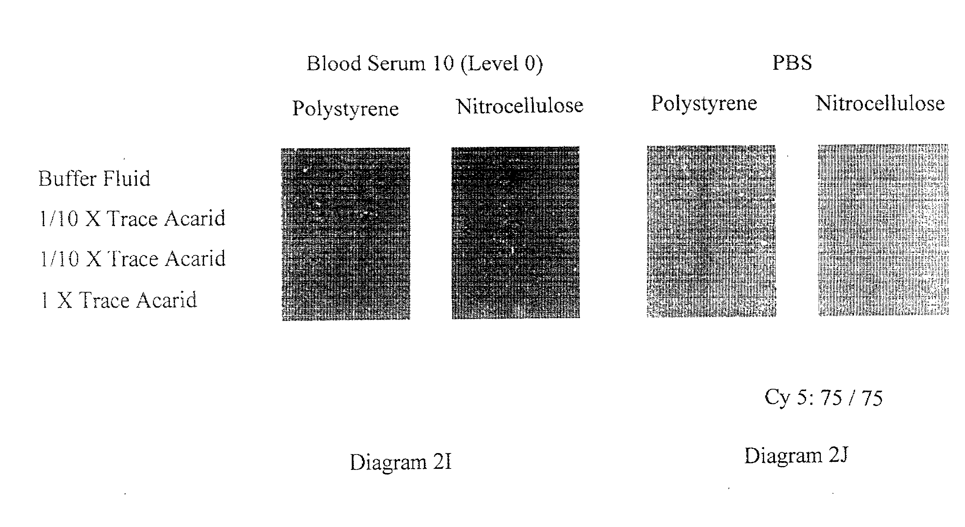

Figs. 3(A)-(D) are the results measured by various allergen chips; Fig. 3A: polystyrene; nitrocellulose; 1 mixed hogweed; buffer solution; 1/10X mixed hogweed; 1/10X mixed hogweed; 1X mixed hogweed; positive control; Fig. 3B: 5 peanut; buffer solution; 1/10X peanut; 1/10X peanut; 1X peanut; positive control; Fig. 3C: 6 shrimp; polystyrene; nitrocellulose; buffer solution; 1/10X shrimp; 1/10X shrimp; 1X shrimp; positive control; Fig. 3D: 7 soya bean; buffer solution; 1/10X soya bean; 1/10X soya bean; 1X soya bean; positive control.

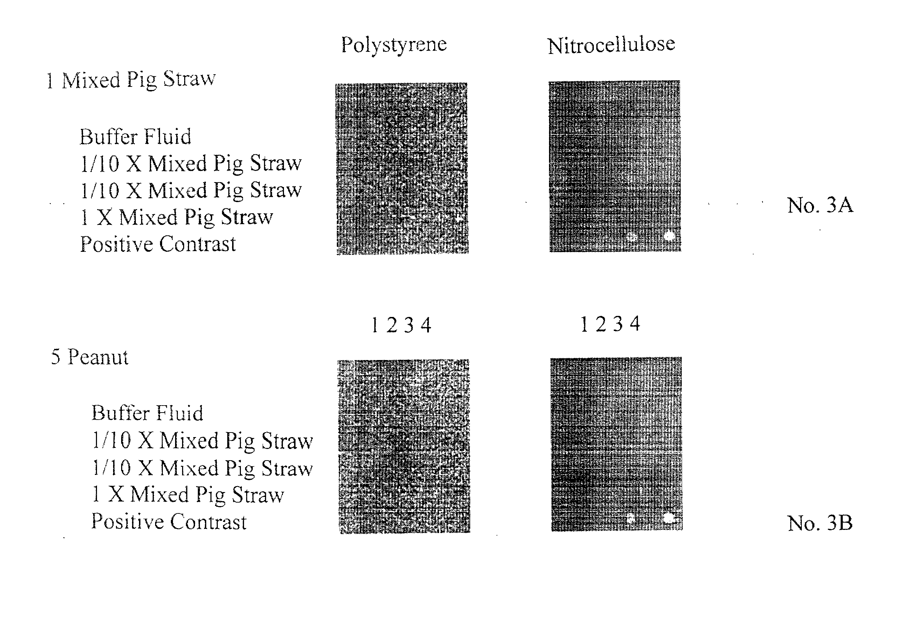

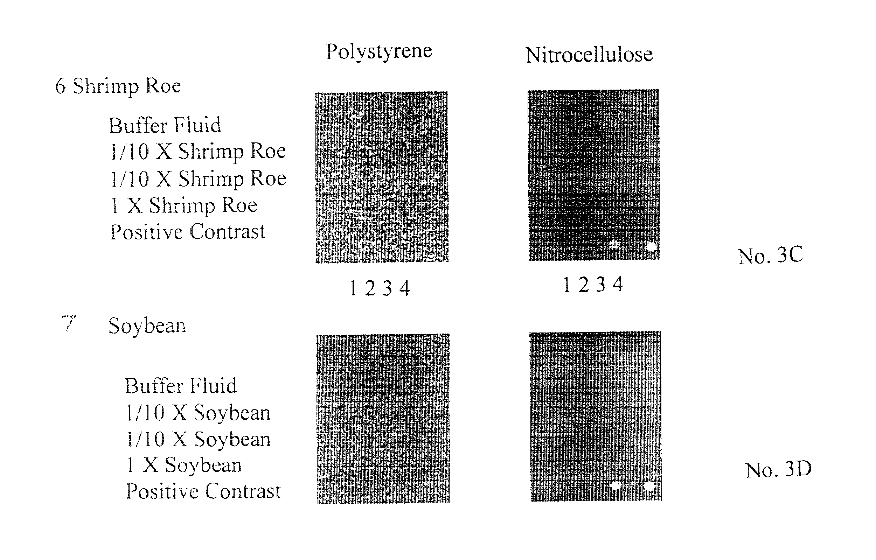

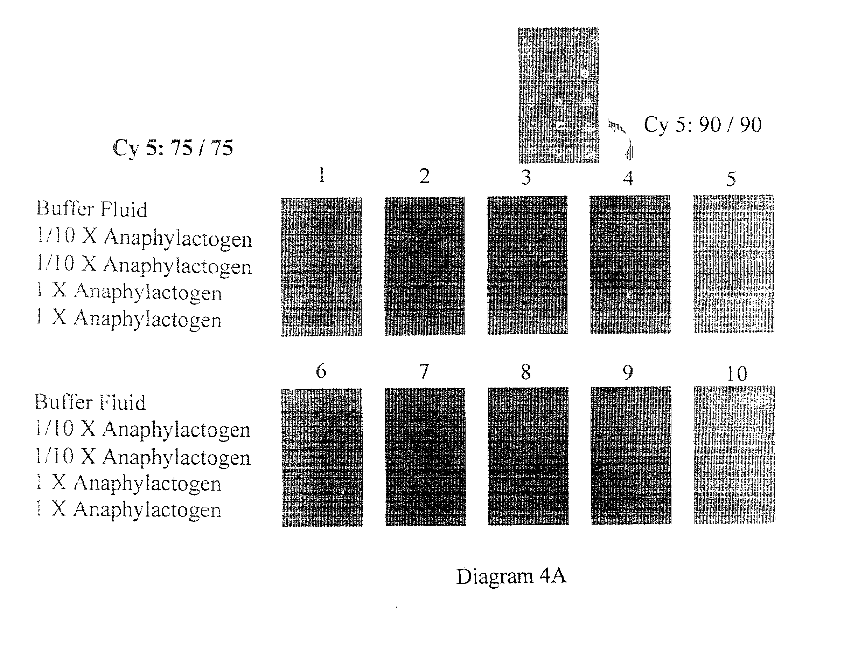

Figs. 4(A)-(B) are the results measured by an allergen chip of milk; Fig. 4A: buffer solution; 1/10X allergen; 1/10X allergen; 1X allergen; 1X allergen; Fig. 4B: buffer solution; 1/10X allergen; 1/10 X allergen; 1X allergen; 1X allergen; test antibody; streptavidin combined with Cy5.

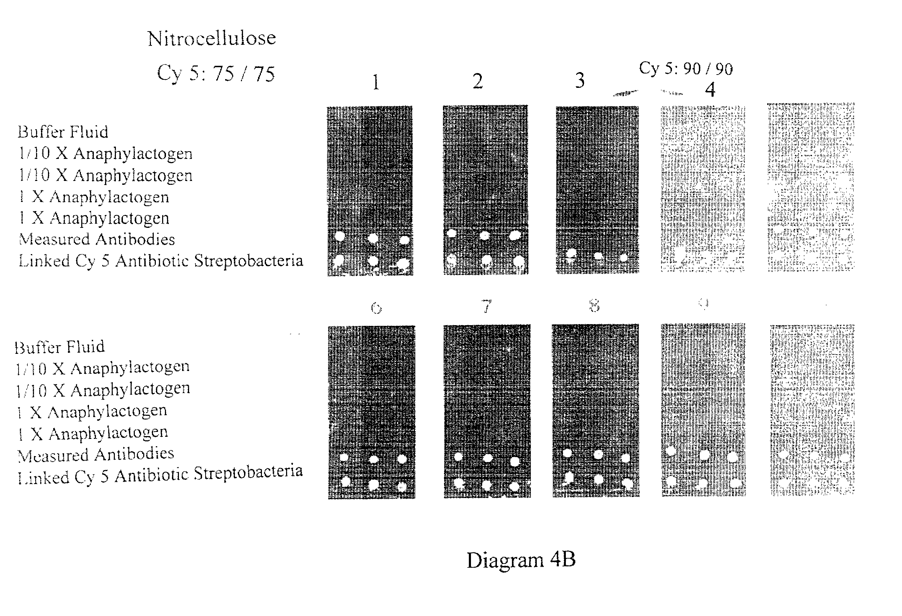

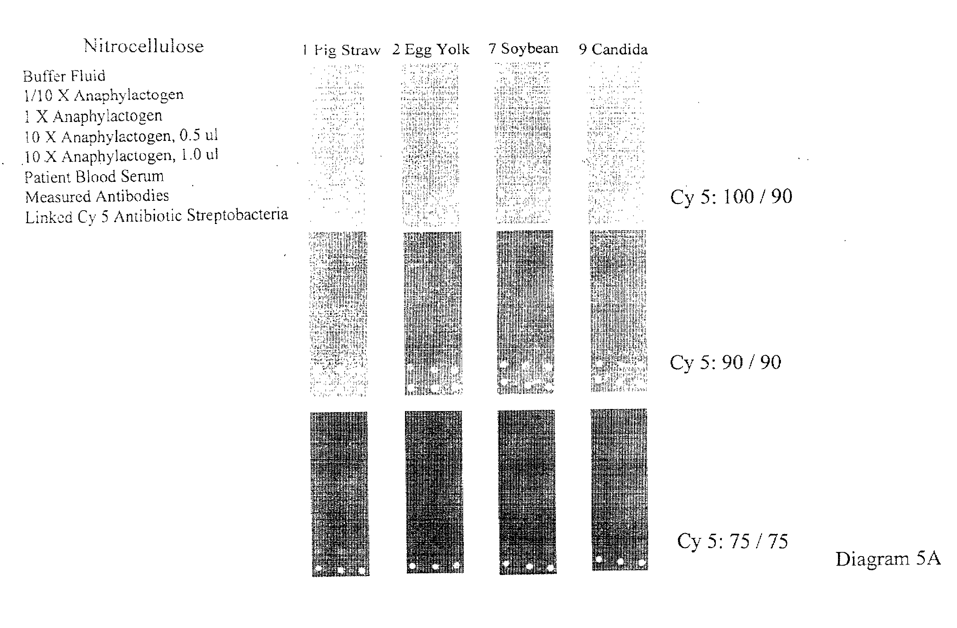

Figs. 5(A)-(B) are the results measured by various allergen chips; Fig. 5A: 1 hogweed; 2 egg yolk; 7 soya bean; 9: Candida albicans; nitrocellulose; buffer solution; 1/10X allergen; 1X allergen; 10X allergen, 0.5ul; 10X allergen, 1.0ul; patient's serum; test antibody; streptavidin combined with Cy5; Fig. 5B: 3 egg white; 5 peanut; 6 shrimp; 7 soya bean; 8 Aspergillus fumigatus; 10 Pencillium notatum; 4 milk; nitrocellulose; buffer solution; 1/10X allergen; 1X allergen; 10X allergen, 0.5ul; 10X allergen, 1.0ul; test antibody; streptavidin combined with Cy5.

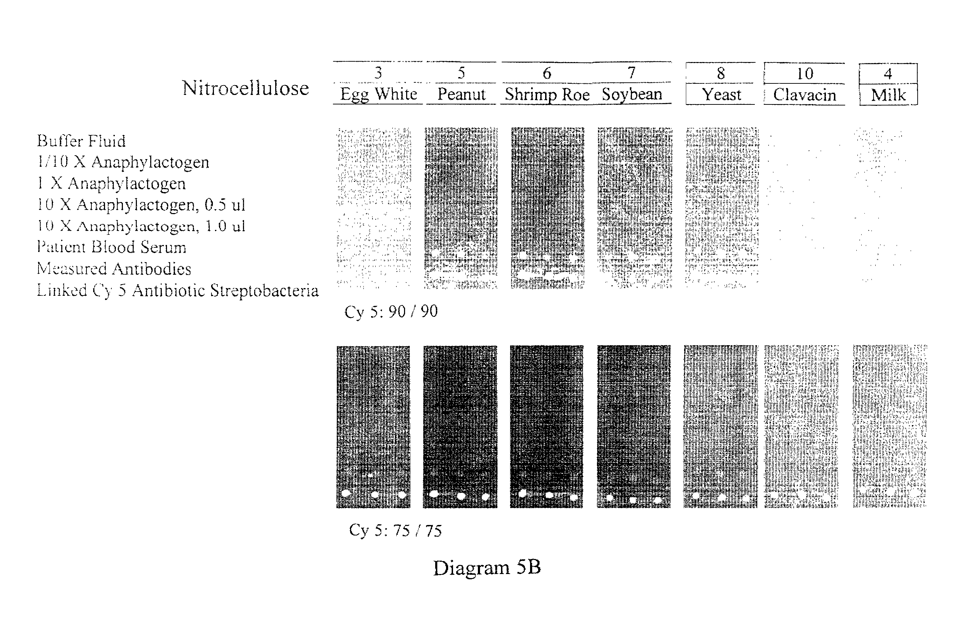

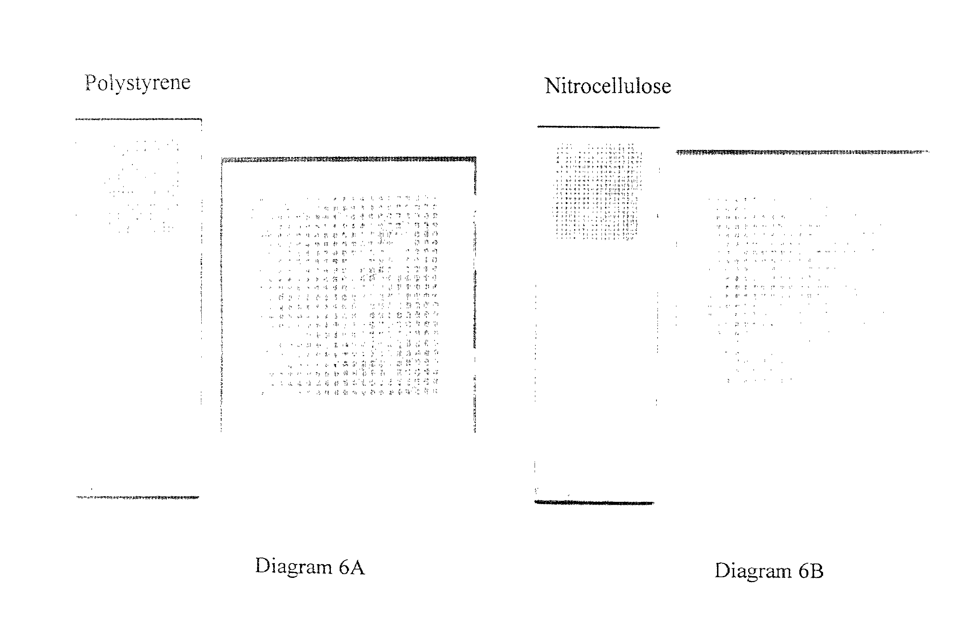

Figs. 6(A)-(B) are high density allergen chips with over 100 allergen spots/cm². Fig. 6A: polystyrene; Fig. 6B: nitrocellulose.

### Legends:

| | |
|---|---|
| 10: allergen chip | 20: solid substrate |
| 30: substrate layer | 40: adhesive intermediate layer |
| 50: surface layer | 60: allergen |

### Detailed Description of the Invention

Referring to Fig. 1, an allergen test chip 10 according to the present invention comprises a solid substrate 20, and at least one allergen 60 fastened to said solid substrate 20, wherein said solid substrate 20 comprises (i) a substrate layer 30; (ii) an adhesive intermediate layer 40 located on said substrate layer 30; and (iii) a surface layer 50 located on said adhesive intermediate layer 40.

Examples of the material suitable as the substrate layer 30 include, but not limited to, glass, silicon chip, ceramic material, metal, polyvinyldifluoride (PVDF) film or cellulose acetate film, etc. The surface layer 50 is used to combine with the allergen; its suitable material includes, but not limited to, nitrocellulose (NC), or polystyrene (PS), etc., preferably nitrocellulose. Furthermore, in order for the surface layer 50 being able to be reliably adhered to the substrate layer 30, preferably an adhesive intermediate layer 40 is used between the two layers. An applicable adhesive intermediate layer includes, but not limited to, an epoxy resin or a chemical material with other similar functional groups.

In another embodiment of the present invention, the solid substrate 20 can be a layer of a material capable of directly combining with the allergen. For example, such a material can be a polymer synthesized from an organic molecule, wherein such an organic molecule includes, but not limited to, styrene, ethylene, propylene, ester, acrylic acid, acrylate, alkyl acrylic acid or alkyl acrylate. In the present invention, examples of the solid substrate capable of directly combining with the allergen include, but not limited to, polystyrene (PS), polyethylene (PE), polypropylene (PP), polycarbonate (PC), etc.

The present invention can detect allergic reactions, including any IgE- and/or IgG-triggered allergic reaction. Therefore, the allergens, that can be fastened to the abovementioned solid substrate, include any matter that can trigger an allergic reaction of a mammal which includes, but not limited to, an extract selected from the group consisting of dust mite, feather, pollen, fungi, bacteria, egg yolk, egg white, hogweed, milk, peanut, shrimp, crab, fish, clam, soybean, mango, cockroach, dog shedding, or cat shedding, etc.

The allergen test chip according to the present invention can be prepared into a chip having a high density, preferably a density of the allergen on the chip of over 100 allergen spots/cm², more preferably over 200, 400, 800, 1600 or more allergen spots/cm². On an allergen test chip according to the present invention, the amount of the sample or test reagent required by each test spot (allergen spot) is smaller than µL, preferably in the order of nanoliter (nL). Such an allergen test chip can effectively fasten various allergens on one chip, thereby achieving the objectives of mass production, versatile, low amount of specimen and low cost.

A method for preparing an allergen test chip according to the present invention comprises coating an adhesive intermediate layer on a substrate layer; coating a surface layer on said adhesive intermediate layer; fastening at least one allergen on said surface layer, wherein said substrate layer, said adhesive intermediate layer, said surface layer, and said allergen(s) are defined as the above. The method of coating is not specifically limited, and can be a process known by the people skilled in the field of chemical engineering and semiconductor process, e.g. spin coating, screen printing, roller coating, curtain coating, dip coating, etc. Preferably, a spin coating process is used to complete the coating of each layer. Prior to the coating of the adhesive intermediate layer, as required, the surface of the substrate layer can be cleaned to remove the impurities or dirt attached to the substrate layer and for the convenience of the adhesion of the adhesive intermediate layer. The abovementioned cleaning step can include the use of a solvent (e.g. surfactant, water, alcohol, acetone, etc.) for cleaning and/or a pretreatment of ultrasonic vibration.

As mentioned in the above, if a solid substrate that can directly combine with the allergen(s) is used, the allergen(s) can be directly fastened to said solid substrate for the preparation of an allergen test chip according to the present invention.

The present invention also provides a kit for the testing of allergen(s), which comprises (i) anyone of the abovementioned allergen test chips; (ii) an isolation solution for isolating a portion of said test chip not in combination with the allergen; (iii) a secondary antibody which can specifically combine with an anti-allergic antibody; (iv) a cleaning solution; and (v) signal generation means for generating a signal by operatively in combination with said secondary antibody.

The term "isolation solution" used herein is used to isolate a portion of said test chip not in combination with the allergen, thereby avoiding a subsequent specimen or a secondary antibody or other matter from attaching to the chip and causing an error in the test. The isolation solution that can be used is known by the people skilled in the art. An ordinary solution of bovine serum albumin (BSA), casein or gelatin all can be used as an isolation solution in the present invention.

The term "secondary antibody" can be a foreign antibody that can specifically combine with the anti-allergic antibody in serum. Such a secondary antibody can include an IgE antibody or an IgG antibody, and can also be a monoclonal antibody or a polyclonal antibody. Generally speaking, a secondary antibody can combine with a constant fragment (Fc) or a variable fragment (Fab) of an anti-allergic antibody. Moreover, these antibodies are commercially available, e.g. Bethyl Laboratories, Inc., TX, U.S.A.; Biogenesis Ltd., England; Jackson Laboratory, Maine, U.S.A., etc.

A "cleaning solution" is used to clean the un-combined residual reagent after the addition of the reagent. Detail of which will be described hereinafter. A cleaning solution suitable for the present invention includes, but not limited to, a phosphate buffer solution (PBS), tris hydroxymethyl amino methane buffer solution (TBS), etc. Optionally, a proteinase inhibitor (e.g. benzamidine) or a surfactant (e.g., NP-40®, Tween-20®, Tween-80®, series, etc.) can be added.

The term "signal generation means" used herein can operatively combine with said secondary antibody, and is known by the people skilled in the art, and can be selectively used according to the need, which, for example, includes, but not limited to, a radioactive marker, a fluorescent marker (e.g., a lanthanum series fluorescent material), a phosphorous marker, luminescent marker (e.g., a bioluminescent marker or a chemiluminescent marker) or an enzyme, etc. Wherein, the useful enzymes include, but not limited to, alkaline phosphorase (AP), hydroperoxidease (HRP) or β -galactosidase, etc. The abovementioned enzymes can be used together with a suitable substrate to change color. The selection of the substrate depends on the selected enzyme, and is known by the people skilled in the art. Suitable substrates include, but not limited to, p-nitrophenolphosphate (pNPP), 2,2'-dinitro-bis-(3-ethylbenzene-thiazolin-6-sulphonic acid (ABTS), 5-bromo-4-chloro-3-indolyl phosphate/nitrobluetetrazoleonium (BCIP/NBT) or naphthol AS-TR phosphate or 3,3',5,5'-tetramethylbenzidine (TMB), etc.

In a preferred embodiment of the present invention, an allergen test kit for testing the allergic reaction can further use a specific combination of a biotin and a streptavidin, thereby achieving the objectives of signal amplification and accuracy elevation. A secondary antibody (e.g., anti-human IgE antibody) is combined with a biotin for identifying an anti-allergic antibody combined to a serum sample. Then, a streptavidin combined with a signal generation means (as abovementioned) is added. Furthermore, a suitable substrate is used for displaying a color. The use of a primary identification by a secondary antibody and an anti-allergen antibody and a secondary specific combination of a biotin to a streptavidin, not only can amplify the test signal, but also can greatly reduce the error.

The present invention also provides an allergen testing method, which comprises (a) providing anyone of the abovementioned allergen test chip; (b) using an isolation solution to isolate a portion of said test chip not in combination with said allergen; (c) contacting the serum sample of a patient with said test chip; (d) using a washing solution to perform a washing step; (e) providing a signal generation means capable of generating a signal by operatively combining with a secondary antibody, wherein said secondary antibody can specifically combine with an anti-allergic antibody; and (f) measuring the signal generated by said signal generation means.

Furthermore, in Step (c), a standard antibody or a positive control or a negative control can be added to build an internal standard curve for quantitative analysis or reference. The use of the abovementioned reagents and the reaction conditions can be referred to Antibody: A Laboratory Manual, Ed. Harlow & Dayid Lane, 1988; the content of which is incorporated herein for reference.

### Examples

The present invention will be further elaborated in greater detail by the following examples. However, these examples are for illustrative purpose only, and are not used to limit the scope of the present invention.

### Example 1: Preparation of an allergen test chip

A glass slide with a suitable size was prepared. The slide was washed with a surfactant, deionized water, acetone, deionized water and alcohol. The washing process was carried out in an ultrasonic oscillator. A solution of an adhesive intermediate layer containing an epoxy resin was coated on the glass slide by a spin coating process. After oven drying, the smoothness and the fluorescent background of the slide were analyzed. A slide with a uniform coating and a low fluorescent background was selected. A spin coating process was used to coat a surface layer (a solution containing nitrocellulose or polystyrene) on the adhesive intermediate layer. After oven drying, a same analysis was used to select a slide with a uniform coating and a low fluorescent background to be used as a solid substrate of the present invention.

Various allergens at a suitable concentration (original concentration or diluted in a volume ratio of 1/10) were dripped on the solid substrate. After loading the allergens, the solid substrate was placed still at 37°C for 1 hour or at 4°C overnight to complete the preparation of an allergen test chip.

### Example 2: Analysis of a dust mite allergen chip

A dust mite allergen (bought from: Center Lab.; Batch No. 9D00242) was separately dripped on a NC or PS solid substrate and placing still overnight at 4°C, and was isolated by a bovine serum albumin (BSA) by placing still overnight at 4°C. The serum antibodies (third or fourth grade) of seven allergic patients from the Tri-Service General Hospital, Taiwan, R.O.C. were added to each slide to be cultivated at 30°C for two hours. Then, an anti-human IgE antibody combined with a biotin (purchased from KPL, serial No. 074-1004) was added and cultivated at 30°C for two hours. Next, a streptavidin combined with a fluorescein (Cy5) was added and cultivated at 30°C for one hour. Then, a scanner was used to detect the fluorescent intensity of each spot. The results were shown in Fig. 2, Table 1A and 1B.

**Table 1A:**

| Analysis of a dust mite allergen chip using nitrocellulose as the surface layer | | | | | | | | | | | | | | |
|---|---|---|---|---|---|---|---|---|---|---|---|---|---|---|
| | S1(fourth grade) | | S2(fourth grade) | | S3(fourth grade) | | S4(fourth grade) | | S5(third grade) | | S6(fourth grade) | | S7(third grade) | |
| | Fluor esce nt value * | S/N | Fluor esce nt value * | S/N | Fluor esce nt value * | S/N | Fluor esce nt value * | S/N | Fluor esce nt value * | S/N | Fluor esce nt value * | S/N | Fluor esce nt value * | S/N |
| 1/10 X dust mite | 1747 | 2.1 | 3554 | 4.0 | 3318 | 7.4 | 6411 | 20.9 | 3279 | 13.1 | 2174 | 8.9 | 3515 | 10 |
| 1/10 X dust mite | - | - | - | - | - | - | 6687 | 21.8 | 4658 | 18.5 | 1725 | 7.1 | 4417 | 12.6 |
| 1X dust mite | - | - | 1935 | 2.2 | 1513 | 3.4 | 3037 | 9.9 | 1813 | 7.2 | 1402 | 5.8 | 3036 | 8.6 |
| Back grou nd value (N) | 839 | - | 902 | - | 448 | - | 308 | - | 252 | - | 244 | - | 353 | - |

| | | | | | | | | | | | | | | |
|---|---|---|---|---|---|---|---|---|---|---|---|---|---|---|
| * Fluorescent value (S) was indicated in an average value. | | | | | | | | | | | | | | |

**Table 1B:**

| Analysis of a dust mite allergen chip using nitrocellulose as the surface layer | | | | | | | | | | | | | | |
|---|---|---|---|---|---|---|---|---|---|---|---|---|---|---|
| | S1(fourth grade) | | S2(fourth grade) | | S3(fourth grade) | | S4(fourth grade) | | S5(third grade) | | S6(fourth grade) | | S7(third grade) | |
| | Fluor esce nt value * | S/N | Fluor esce nt value * | S/N | Fluor esce nt value * | S/N | Fluor esce nt value * | S/N | Fluor esce nt value * | S/N | Fluor esce nt value * | S/N | Fluor esce nt value * | S/N |
| 1/10 X dust mite | - | - | - | - | - | - | 793 | 2.0 | 1904 | 2.0 | 1563 | 1.2 | 2824 | 3.4 |
| 1/10 X dust mite | - | - | - | - | - | - | 1333 | 3.3 | 1481 | 1.6 | 1907 | 1.4 | 2784 | 3.3 |
| 1X dust mite | - | - | - | - | - | - | - | - | 1037 | 1.1 | 1463 | 1.1 | 1278 | 1.5 |
| Back grou nd value (N) | - | - | - | - | - | - | 407 | - | 957 | - | 1402 | - | 852 | - |

| | | | | | | | | | | | | | | |
|---|---|---|---|---|---|---|---|---|---|---|---|---|---|---|
| * Fluorescent value (S) was indicated in an average value. | | | | | | | | | | | | | | |

### Example 3: Analysis of various allergen chips (1)

Allergens of hogweed (1), peanut (2), shrimp (3) and soybean (4) at various concentration (original concentration or diluted in a volume ratio of 1/10) were dripped on a solid substrate using NC or PS as the surface layer. Serum antibodies purchased from AlerCHEK (batch No. 210901, 106801 and 109901) were used as the test samples. The rest of the steps were identical to Example 2. The results were shown in Fig. 3 wherein the "positive control" indicated the result where an allergen with an original concentration (un-diluted) was dripped, a patient's serum was used as a sample, a goat anti-human IgE antibody combined with a biotin was used as a secondary antibody, and a streptavidin combined with Cy5 was used for reaction.

### Example 4: Analysis of milk allergen chip

A milk was used as an allergen and dripped on a solid substrate with NC as the surface layer. A serum antibody from an allergic patient (third grade) from the National Taiwan University Hospital, Taiwan, R.O.C. was used as the test specimen. The rest of the steps were the same as Example 2. The results were shown in Fig. 4 and Table 2. In Fig. 4, the "test antibody" was prepared by dripping a secondary antibody on a chip, after washing, adding a streptavidin combined with Cy5 for testing the reaction result; and the "streptavidin combined with Cy5" was prepared by directly dripping a streptavidin on a chip, after washing, for testing the reaction result. Both could be used as a positive control of the present example.

**Table 2:**

| Analysis of milk allergen chip using nitrocellulose as the surface layer | | | | |
|---|---|---|---|---|
| | Patient A | | Patient A1 | |
| | Fluorescent value (S)* | S/N S/N | Fluorescent value (S)* | S/N |
| 1/10X allergen | 6203 | 2.0 | 4953 | 1.5 |
| 1/10X allergen | 7945 | 2.6 | 5184 | 1.6 |
| 1X allergen | 6127 | 2.0 | 4354 | 1.3 |
| 1X allergen | 5648 | 1.8 | 4658 | 1.4 |
| Background value (N) | 3090 | - | 3408 | - |

| | | | | |
|---|---|---|---|---|
| * Fluorescent value (S) was indicated in an average value. | | | | |

### Example 5: Analysis of various allergen chips (2)

In the present example, allergens of hogweed (1), egg yolk (2), egg white (3), milk (4), peanut (5), shrimp (6), soya bean (7), Aspergillus fumigatus (8), Candida albicans (9), and Pencillium notatum (10) at various concentration (original concentration, diluted in a volume ratio of 1/10, or concentrated for ten times) were separately dripped on a solid substrate using NC as the surface layer. A serum antibody from an allergic patient (third grade) from the National Taiwan University Hospital, Taiwan, R.O.C. was used as the test specimen. The rest of the steps are the same as Example 2. The results were shown in Fig. 5 and Table 3, wherein the "test antibody" and the "streptavidin combined with Cy5" are the same as Ex. 4.

**Table 3:**

| Analysis of various allergen chips using nitrocellulose as the surface layer | | | | | | | | |
|---|---|---|---|---|---|---|---|---|
| | Egg yolk | | Candida albicans | | Egg white | | Milk | |
| | Fluoresc ent value * | S/N | Fluoresc ent value * | S/N | Fluoresc ent value * | S/N | Fluoresc ent value * | S/N |
| 1/10 X allergen | 16920 | 1.8 | - | - | 13623 | 1.2 | 9890 | 1.7 |
| 1X allergen | 12831 | 1.4 | 11622 | 1.2 | 13691 | 1.2 | 10020 | 1.7 |
| 10X allergen, 0.5 µl | 11688 | 1.2 | 11851 | 1.3 | 18639 | 1.6 | 9892 | 1.7 |
| 10X allergen, 1.0 µl | 12535 | 1.3 | 13324 | 1.4 | 19406 | 1.7 | 8911 | 1.5 |
| Serum of patient | - | - | - | - | - | - | - | - |
| Anti-hum an IgE antibody-biotin | 30175 | 3.2 | 25831 | 2.7 | 34233 | 2.9 | 15763 | 2.7 |
| Streptavi din combined with Cy5 | 65280 | 6.8 | 63525 | 6.5 | 65280 | 5.5 | 63841 | 10.8 |
| Backgrou nd value (N) | 9687 | - | 9776 | - | 11798 | - | 5923 | - |

| | | | | | | | | |
|---|---|---|---|---|---|---|---|---|
| * Fluorescent value (S) was indicated in an average value. | | | | | | | | |

### Example 6: Test of density specification for allergen test chip

A soya bean solution of 0.1 g/ml was dripped to an allergen test chip (nitrocellulose and polystyrene) prepared in Example 1 by a dot matrix machine. The dot matrix density was 484 dots/4.41 cm² (about 110 dots/cm²). After fastening, a Coomassie Blue dye was used to identify the performance of each dot. The results were shown in Fig. 6.

In the above analysis, the grade of allergen was classified according to the standard stipulated in the MAST-CLA test (Hitachi Chemical Diagnosis Co.). Furthermore, the ratio (S/N) of the fluorescent value/background value measured by the present invention was used to determine the existence of an antibody of an allergen in a specimen under test. The present invention defined that, when a ratio of S/N was greater than 2, a positive allergic reaction could be preliminarily determined. On the other hand, when a ratio was smaller than 2, a negative reaction was determined.

From the above results, an allergen test chip according to the present invention can effectively fasten various allergens on a same chip. Therefore, the type and concentration of an allergen, which triggers a patient's allergic reaction, can be tested in a large quantity, a low amount of specimen used and at a low cost. The test results can be used for a subsequent processing and treatment by a specialist. Furthermore, an allergen test chip according to the present invention can have a high sensitivity and accuracy in the test results through the specific reaction of a biotin-streptavidin, or through combining with a signal generation means with a high sensitivity.

Although the present invention has been disclosed in the abovementioned preferred examples, the examples are not used to limit the scope of the present invention. Any person skilled in the art can perform various changes and modifications without departure from the spirit and scope of the present invention. For example, other types of allergen, signal generation means combined with a secondary antibody and the modification and improvement of various detection methods, are all within the scope of the present invention. Therefore, the scope of the present invention is defined by the appended claims.

## Claims

1. An allergen test chip, which comprises:
(i) a solid substrate; and
(ii) at least one allergen fastened to said solid substrate.

2. An allergen test chip according to Claim 1 wherein said solid substrate comprises:
(i) a substrate layer;
(ii) an adhesive intermediate layer located on said substrate layer; and
(iii) a surface layer located on said adhesive intermediate layer.

3. The allergen test chip as claimed in Claim 2, wherein said adhesive intermediate layer comprises an epoxy resin.

4. The allergen test chip as claimed in Claims 2 or 3, wherein said surface layer comprises nitrocellulose or polystyrene.

5. The allergen test chip as claimed in any one of Claims 1 to 4, wherein said solid substrate comprises a polymer synthesized from an organic molecule, wherein said organic molecule is selected from a group consisting of styrene, ethylene, propylene, ester, acrylic acid, acrylate, alkyl acrylic acid and alkyl acrylate.

6. The allergen test chip as claimed in any one of Claims 1 to 4, wherein said substrate layer comprises glass, a silicon chip, a ceramic material, a metal, a polyvinyldifluoride (PVDF) film or a cellulose acetate film.

7. The allergen test chip as claimed in any one of Claims 1 to 6, wherein said allergen is an extract selected from dust mite, feather, pollen, fungi, bacteria, egg yolk, egg white, hogweed, milk, peanut, shrimp, crab, fish, clam, soybean, mango, cockroach, dog shedding, and cat shedding.

8. The allergen test chip as claimed in any one of Claims 1 to 7 wherein the allergen density on said allergen test chip is greater than 100 allergens/cm².

9. A method for preparing an allergen test chip, which comprises
(a) coating an adhesive intermediate layer on a substrate layer;
(b) coating a surface layer on said adhesive intermediate layer; and
(c) fastening at least one allergen to said surface layer.

10. The method for preparing an allergen test chip as claimed in Claim 10, wherein said test chip is in accordance with any one of Claims 1 to 10.

11. The method for preparing an allergen test chip as claimed in Claim 9 or 10, wherein said coating method comprises spin coating, dip coating, screen printing, roller coating, or curtain coating.

12. The method for preparing an allergen test chip as claimed in Claim 11, wherein said coating method is spin coating.

13. The method for preparing an allergen test chip as claimed in any one of Claims 1 to 12 which, prior to coating said adhesive intermediate layer, further comprises a step of cleaning the surface of said substrate layer.

14. The method for preparing an allergen test chip as claimed in Claim 13, wherein said cleaning step is a pretreatment of cleaning with a solvent and/or an ultrasonic oscillation.

15. The method for preparing an allergen test chip as claimed in Claim 14, wherein said solvent is selected from surfactant, water, alcohol and acetone.

16. A method for preparing an allergen test chip, which comprises fastening at least one allergen to a solid substrate.

17. A method for preparing an allergen test chip wherein the test chip is in accordance with any one of Claims 1 to 10.

18. A kit for testing an allergen, which comprises:
(i) an allergen test chip as claimed in any one of Claims 1 to 10;
(ii) an isolation solution for isolating a portion of said test chip not in combination with the allergen;
(iii) a secondary antibody which can specifically combine with an anti-allergic antibody;
(iv) a cleaning solution; and
(v) signal generation means for generating a signal by operatively in combination with said secondary antibody.

19. The kit as claimed in Claim 18, wherein said secondary antibody comprises an anti-IgE antibody or an anti-IgG antibody.

20. The kit as claimed in Claim 19 wherein said antibody comprises a monoclonal antibody or a polyclonal antibody.

21. The kit as claimed in any one of Claims 18 to 20 wherein said cleaning solution comprises a phosphate buffer solution (PBS) or a tris hydroxymethyl amino methane buffer solution (TBS).

22. The kit as claimed in Claim 21, wherein said cleaning solution further comprises a surfactant.

23. The kit as claimed in any one of Claims 18 to 22, wherein said signal generation means is selected from a group consisting of a radioactive marker, a fluorescent marker, a phosphorous marker, a luminescent marker, and an enzyme.

24. The kit as claimed in Claim 23, wherein said luminescent marker comprises a bioluminescent marker or a chemiluminescent marker.

25. The kit as claimed in Claim 23, wherein said enzyme is selected from an alkaline phosphorase (AP), a hydroperoxidease (HRP) and a β-galactosidase.

26. The kit as claimed in Claim 25, which further comprises a substrate, wherein said substrate can react with said enzyme and display a color.

27. The kit as claimed in any one of Claims 23 to 26, wherein said signal generation means further comprise a biotin.

28. The kit as claimed in Claim 27, which further comprises an anti-biotin protein which can operatively combine with a radioactive marker, a fluorescent marker, a phosphorous marker, a luminescent marker, or an enzyme.

29. The kit as claimed in any one of Claims 18 to 22, wherein said isolation solution comprises a solution of bovine serum albumin (BSA), casein or gelatin.

30. A method for testing an allergen, which comprises:
(a) providing an allergen test chip as claimed in any one of Claims 1 to 10;
(b) using an isolation solution to isolate a portion of said test chip not in combination with said allergen;
(c) contacting the serum sample of a patient with said test chip;
(d) using a washing solution to perform a washing step;
(e) providing a signal generation means capable of generating a signal by operatively combining with a secondary antibody, wherein said secondary antibody can specifically combine with an anti-allergic antibody; and
(f) measuring the signal generated by said signal generation means.

31. The method as claimed in Claim 30, wherein said secondary antibody comprises an anti-IgE antibody or an anti-IgG antibody.

32. The method as claimed in Claim 31, wherein said antibody comprises a monoclonal antibody or a polyclonal antibody.

33. The method as claimed in any one of Claims 30 to 32, wherein Step (c) further comprises contacting a control standard antibody with said test chip.

34. The method as claimed in any one of Claims 30 to 33, wherein said cleaning solution comprises a phosphate buffer solution (PBS) or a tris hydroxymethyl amino methane buffer solution (TBS).

35. The method as claimed in Claim 34, wherein said cleaning solution further comprises a surfactant.

36. The method as claimed in any one of Claims 30 to 35, wherein said signal generation means is a radioactive marker, a fluorescent marker, a phosphorous marker, a luminescent marker, or an enzyme.

37. The method as claimed in Claim 36, wherein said luminescent marker comprises a bioluminescent marker or a chemiluminescent marker.

38. The method as claimed in Claim 36, wherein said enzyme is selected from a group consisting of an alkaline phosphorase (AP), a hydroperoxidease (HRP) and a β-galactosidase.

39. The method as claimed in any one of Claims 30 to 38, which further comprises adding a substrate, wherein said substrate can react with said enzyme and display a color.

40. The method as claimed in Claim 36, wherein said signal generation means further comprises a biotin.

41. The method as claimed in Claim 40, which further comprises adding an anti-biotin protein which can operatively combine with a radioactive marker, a fluorescent marker, a phosphorous marker, a luminescent marker, or an enzyme.

42. The method as claimed in Claim 41, wherein said enzyme is selected from an alkaline phosphorase (AP), a hydroperoxidease (HRP) and a β-galactosidase.

43. The method as claimed in any one of Claims 30 to 42, wherein said isolation solution comprises a solution of bovine serum albumin (BSA), casein or gelatin.
